# EUROPEAN PATENT APPLICATION

(11) **EP 3 384 832 A1**
(43) Date of publication of application: **10.10.2018**
(21) Application number: 17165224.1
(22) Date of filing: 06.04.2017
(51) Int. Cl.: A61B 5/00

(54) **METHOD AND APPARATUS FOR PROVIDING GUIDANCE FOR PLACEMENT OF A WEARABLE DEVICE**

(71) Applicant: Koninklijke Philips N.V., 5656 AE Eindhoven (NL)
(72) Inventor: TALGORN, Elise Claude Valentine, 5656 AE Eindhoven (NL); GEURTS, Lucas Jacobus Franciscus, 5656 AE Eindhoven (NL); BUIL, Vincentius Paulus, 5656 AE Eindhoven (NL); MEFTAH, Mohammed, 5656 AE Eindhoven (NL); PASTOOR, Sander Theodoor, 5656 AE Eindhoven (NL)
(74) Representative: de Haan, Poul Erik

(57) **Abstract**

There is provided a method and apparatus comprising a processor to provide guidance for placement of a wearable device. At least one image of the body of a subject is acquired from one or more cameras (502). The at least one acquired image is analysed to recognise body parts of the subject and to identify a body part of the subject at which to place the wearable device (504). Guidance to place the wearable device at the identified body part of the subject is provided (506).

## Description

### Technical Field of the Invention

The invention relates to the field of wearable devices and, in particular, to a method and apparatus for providing guidance for placement of a wearable device.

### Background to the Invention

Wearable devices and, in particular, wearable sensors or wearable medication dispensers (such as sensor patches or medication dispenser patches) play a pivotal role in medical care and future rehabilitation procedures. Often sensors worn by a subject form part of a body area network through which medical professionals can acquire data on the subject from a remote location. The data can, for example, include the vital signs of the subject. The wearable sensors are usually placed on the body of the subject at a location that is appropriate for the relevant information to be acquired. Similarly, wearable medication dispensers are usually placed on the body of the subject at a location that is appropriate for the medication to be given. For this reason, the placement of such wearable devices is typically done by a medical professional (such as a nurse) in a medical environment (such as a hospital).

However, wearable devices are now being used in a wider variety of situations. For example, wearable sensors can be used for monitoring subjects in low acuity settings (such as in a general ward or at home) and can even be used by subjects to monitor themselves. There is an increased need to use sensors in low acuity settings, which is emphasised by the demand for improved monitoring in general wards to detect deterioration of subjects as early as possible (and thus reduce mortality rates) and also by the growing need to discharge subjects earlier, whilst still continuing a level of monitoring at home.

Most wearable devices need to be replaced every few days due to battery depletion, hygiene, degradation of adhesives, or skin irritation. As a result, the subjects themselves or informal caregivers often need to replace the wearable device. A difficulty is that the placement of the wearable devices at a correct location on the body of a subject is often key for the performance and/or the proper operation of the wearable devices. In particular, for example, a wearable sensor in the form of an electrocardiography (ECG) patch needs to be placed at an accurate location on the chest of the subject. However, placing wearable devices at a correct location can be challenging. This is especially the case for an untrained user, particularly where the user is elderly as the user may have problems with eyesight, dexterity, bending, or other issues.

There already exist methods for providing guidance for placement of a sensor that can help in sensor replacement. For example, WO 2015/015385 A1 discloses that images acquired from a camera can be analysed for providing guidance for placement of a sensor. Specifically, the images are analysed to identify markers that are attached to anatomical locations of the subject and the sensor is guided to a desired location based on a spatial relationship between these anatomical locations and the desired location.

However, there is still a need for a more accurate and more personalised method for facilitating placement of a wearable device at a correct location on the body of a subject. It would also be valuable to provide a more integrated system for placement of a wearable device that does not need to rely on physical markers attached to the body of the subject.

Therefore, an improved method and apparatus for providing guidance for placement of a wearable device is required.

### Summary of the Invention

As noted above, it would be valuable to have an improved method and apparatus for providing guidance for placement of a wearable device, which overcome existing problems.

Therefore, according to a first aspect of the invention, there is provided a method of operating an apparatus comprising a processor to provide guidance for placement of a wearable device. The method comprises acquiring at least one image of the body of a subject from one or more cameras, analysing the at least one acquired image to recognise body parts of the subject and to identify a body part of the subject at which to place the wearable device, and providing guidance to place the wearable device at the identified body part of the subject.

In some embodiments, the identified body part may be specific to a purpose for which the wearable device is dedicated. In some embodiments, the identified body part may be a body part that is predefined by a user. In some embodiments, the body part at which to place the wearable device may be identified using a skeleton recognition technique.

In some embodiments, the method may further comprise tracking the identified body part in the at least one image as the wearable device approaches the identified body part and adjusting the guidance provided based on the tracking.

In some embodiments, the method may further comprise detecting a location of the wearable device in relation to the identified body part as the wearable device approaches the identified body part and the guidance provided to place the wearable device at the identified body part may comprise guidance to adjust the location of the wearable device in relation to the identified body part. In some embodiments, the method may further comprise detecting an orientation of the wearable device in relation to the identified body part as the wearable device approaches the identified body part and the guidance provided to place the wearable device at the identified body part may comprise guidance to adjust the orientation of the wearable device in relation to the identified body part.

In some embodiments, the method may further comprise acquiring information on a proximity of the wearable device to the identified body part as the wearable device approaches the identified body part. In some embodiments, the method may further comprise, when the proximity of the wearable device to the identified body part is equal to or less than a proximity threshold, identifying at least one marker on the identified body part in the at least one acquired image, tracking the at least one marker on identified body part in the at least one image as the wearable device approaches the identified body part and adjusting the guidance provided based on the tracking.

According to a second aspect of the invention, there is provided a computer program product comprising a computer readable medium, the computer readable medium having computer readable code embodied therein, the computer readable code being configured such that, on execution by a suitable computer or processor, the computer or processor is caused to perform the method or the methods described above.

According to a third aspect of the invention, there is provided an apparatus for providing guidance for placement of a wearable device. The apparatus comprises a processor configured to acquire at least one image of the body of the subject from one or more cameras, analyse the at least one acquired image to recognise body parts of the subject and to identify a body part of the subject at which to place the wearable device, and provide guidance to place the wearable device at the identified body part of the subject.

In some embodiments, one or more cameras may be aimed directly at the body of the subject, one or more cameras may be aimed indirectly at the body of the subject via a reflective surface, or one or more cameras may be aimed directly at the body of the subject and one or more cameras may be aimed indirectly at the body of the subject via a reflective surface. In some embodiments, the wearable device may comprise at least one of the one or more cameras or a mobile device may comprise at least one of the one or more cameras. In some embodiments, the mobile device may comprise an attachment configured to hold the wearable device for the placement.

In some embodiments, the processor may be configured to control a user interface to provide the guidance.

According to the aspects and embodiments described above, the limitations of existing techniques are addressed. In particular, according to the above-described aspects and embodiments, it is possible to simply and accurately facilitate placement of a wearable device at a correct location on the body of a subject, irrespective of the unique anatomy of the subject. Also, a more integrated system for placement of a wearable device is provided that does not need to rely on physical markers attached to the body of the subject. There is thus provided an improved method and apparatus for providing guidance for wearable device placement, which overcomes existing problems.

### Brief Description of the Drawings

For a better understanding of the invention, and to show more clearly how it may be carried into effect, reference will now be made, by way of example only, to the accompanying drawings, in which:
Figure 1 is a block diagram of an apparatus according to an embodiment;
Figure 2 is an illustration of an apparatus according to an example embodiment;
Figure 3 is an illustration of an apparatus in use according to an example embodiment;
Figure 4 is an illustration of an apparatus in use according to another example embodiment;
Figure 5 is a flow chart illustrating a method according to an embodiment;
Figure 6 is a flow chart illustrating a method according to an example embodiment;
Figure 7 is a flow chart illustrating a method according to another example embodiment; and
Figure 8 is a flow chart illustrating a method according to another example embodiment.

### Detailed Description of the Preferred Embodiments

As noted above, the invention provides a method and apparatus for providing guidance for placement of a wearable device, which overcomes the existing problems.

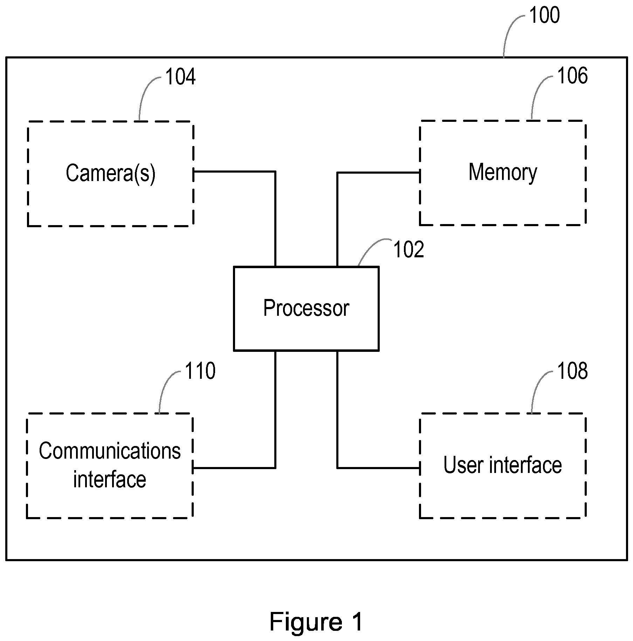

**Figure 1** shows a block diagram of an apparatus 100 according to an embodiment that can be used for providing guidance for placement of a wearable device. Specifically, the apparatus 100 can be used in providing guidance for placement of a wearable device on a part of the body of a subject. The subject may, for example, be a patient, a user, or any other subject. In some embodiments, the apparatus 100 may be a device dedicated for the purpose of providing guidance for wearable device placement. In other embodiments, the apparatus 100 may be a device having other functionalities. For example, in some embodiments, the apparatus 100 may be a mobile device such as a smart phone, a tablet, a laptop, or any other mobile device.

With reference to Figure 1, the apparatus 100 comprises a processor 102 that controls the operation of the apparatus 100 and that can implement the method described herein. The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method according to embodiments of the invention.

Briefly, the processor 102 of the apparatus 100 is configured to acquire at least one image of the body of the subject from one or more cameras, analyse the at least one acquired image to recognise body parts of the subject and to identify a body part of the subject at which to place the wearable device, and provide guidance to place the wearable device at the identified body part of the subject.

The wearable device can be any device that is adapted to be worn by a user (i.e. any wearable device). In some embodiments, for example, the wearable device may be in the form of a patch. The wearable device may comprise an adhesive surface for adhering to the skin of the subject. However, while some example forms of wearable device have been provided, it will be understood that any other forms of wearable device are also possible.

In some embodiments, the wearable device may be a wearable medication dispenser. The wearable medication dispenser can be any wearable medication dispenser for dispensing (or delivering) a medication to the subject. Alternatively or in addition, in some embodiments, the wearable device may be a wearable sensor. The wearable sensor may be a sensor for monitoring the health of the subject. According to some embodiments, the sensor may comprise one or more measurement sensors configured to acquire one or more signals from a subject. The signals may, for example, comprise measurement data.

For example, the sensor may comprise at least one physiological characteristic (or vital signs) sensor. Examples of a physiological characteristic sensor include, but are not limited to, a heart rate sensor configured to acquire a signal indicative of a heart rate of the subject, a heart rate variability sensor configured to acquire a signal indicative of a heart rate variability of the subject, a blood pressure sensor configured to acquire a signal indicative of a blood pressure of the subject, a skin conductance sensor configured to acquire a signal indicative of a skin conductance response of the subject, a skin temperature sensor configured to acquire a signal indicative of a skin temperature of the subject, or any other physiological characteristic sensor, or any combination of physiological characteristic sensor.

Alternatively or in addition to at least one physiological characteristic sensor, the sensor may comprise at least one motion sensor configured to acquire motion information for the subject. Examples of a motion sensor include, but are not limited to an accelerometer, a gravity sensor, an inertial sensor, a gyroscope, a magnetometer, one or more cameras (such as one or more depth sensing cameras), a sensor that employs a computer vision based registration technique, a sensor that employs a radio or acoustics based localisation and orientation technique, or any other motion sensor, or any combination of motion sensors.

Although examples have been provided for the types of sensor, it will be understood that any other types of sensor or any combinations of sensors are also possible. Also, although examples of a wearable sensor and a wearable medication dispenser have been provided for the wearable device, it will be understood that the apparatus and method disclosed herein can be used in respect of any other type of wearable device.

As mentioned earlier, the processor 102 of the apparatus 100 is configured to acquire at least one image of the body of the subject from one or more cameras 104. In some embodiments, the processor 102 of the apparatus 100 can be configured to control the one or more cameras 104 to acquire the at least one acquired image of the body of the subject. As illustrated in Figure 1, in some embodiments, the apparatus 100 may comprise one or more cameras 104 from which at least one image of the body of the subject can be acquired. Alternatively or in addition, one or more cameras 104 from which at least one image of the body of the subject can be acquired may be external to (i.e. separate to or remote from) the apparatus 100. For example, one or more cameras 104 may be part of another device. In some embodiments, the wearable device itself can comprise at least one of the one or more cameras 104.

Alternatively or in addition, a mobile device can comprise at least one of the one or more cameras 104. In some embodiments, the one or more cameras 104 may comprise a front camera of the mobile device, a back camera of the mobile device, or both a front camera and a back camera of the mobile device. As mentioned earlier, the apparatus 100 may be a mobile device according to some embodiments. Thus, the mobile device comprising at least one of the one or more cameras 104 can be the apparatus 100 itself, another mobile device, or both the apparatus 100 and another mobile device may comprise at least one of the one or more cameras 104 according to some embodiments. According to some embodiments, one or more cameras 104 may be aimed directly at the body of the subject. Alternatively or in addition, according some embodiments, one or more cameras 104 may be aimed indirectly at the body of the subject via a reflective surface (such as a mirror, a smart mirror, or any other reflective surface). In some embodiments, different camera lenses (such as a fish eye lens, or any other lens) may be applied to one or more cameras 104.

As illustrated in Figure 1, in some embodiments, the apparatus 100 may also comprise a memory 106. The memory 106 of the apparatus 100 can be configured to store program code that can be executed by the processor 102 to perform the method described herein. Alternatively or in addition to the memory 106 of the apparatus 100, one or more memories 106 may be external to (i.e. separate to or remote from) the apparatus 100. For example, one or more memories 106 may be part of another device. A memory 106 can be used to store information, data, signals and measurements that are acquired or made by the processor 102 of the apparatus 100 or from any components, units, interfaces, sensors, memories, or devices that are external to the apparatus 100. The processor 102 may be configured to control a memory 106 to store information, data, signals and measurements resulting from the method disclosed herein.

According to some embodiments, the apparatus 100 may also comprise at least one user interface 108. Alternatively or in addition, a user interface 108 may be external to (i.e. separate to or remote from) the apparatus 100. For example, a user interface 108 may be part of another device. A user interface 108 may be for use in providing a user with information resulting from the method according to the invention. The user may be the subject themselves, a medical professional, a carer, a family member, or any other user. The processor 102 may be configured to control one or more user interfaces 108 to provide information resulting from the method according to the invention. For example, in some embodiments, the processor 102 may be configured to control one or more user interfaces 108 to render (or output or provide) the guidance for wearable device placement. A user interface 108 may, alternatively or in addition, be configured to receive a user input. In other words, a user interface 108 may allow the user of the apparatus 100 to manually enter data, instructions, or information. The processor 102 may be configured to acquire the user input from one or more user interfaces 108.

A user interface 108 may be any user interface that enables rendering (or outputting) of information, data or signals to a user of the apparatus 100. Alternatively or in addition, a user interface 108 may be any user interface that enables a user of the apparatus 100 to provide a user input, interact with and/or control the apparatus 100. For example, the user interface 108 may comprise one or more switches, one or more buttons, a keypad, a keyboard, a mouse, a touch screen or an application (for example, on a smart device such as a tablet, a smartphone, or any other smart device), a display or display screen, a graphical user interface (GUI) or any other visual component, one or more speakers, one or more microphones or any other audio component, one or more lights (such as light emitting diode LED lights), a component for providing tactile or haptic feedback (such as a vibration function, or any other tactile feedback component), an augmented reality device (such as augmented reality glasses, or any other augmented reality device), a smart device (such as a smart mirror, a tablet, a smart phone, a smart watch, or any other smart device), or any other user interface, or combination of user interfaces. In some embodiments, the user interface that is controlled to render (or output or provide) information, data or signals of the apparatus 100 may be the same user interface as that which enables the user to provide a user input, interact with and/or control the apparatus 100.

As illustrated in Figure 1, in some embodiments, the apparatus 100 may also comprise a communications interface (or circuitry) 110 for enabling the apparatus 100 to communicate with (or connect to) any components, interfaces, units, memories, sensors and devices that are internal or external to the apparatus 100. The communications interface 110 may communicate with any components, interfaces, units, sensors and devices wirelessly or via a wired connection. For example, in embodiments where one or more memories 106 are external to the apparatus 100, the communications interface 110 may communicate with the external memories wirelessly or via a wired connection. Similarly, in the embodiments where one or more user interfaces 108 are external to the apparatus 100, the communications interface 110 may communicate with the external user interfaces wirelessly or via a wired connection.

It will be appreciated that Figure 1 only shows the components required to illustrate this aspect of the invention, and in a practical implementation the apparatus 100 may comprise additional components to those shown. For example, the apparatus 100 may comprise a battery or other power supply for powering the apparatus 100 or means for connecting the apparatus 100 to a mains power supply.

According to some embodiments, the apparatus 100 can comprise an attachment (such as a holder or a connector) configured to hold (or receive or connect to) the wearable device for placement of the wearable device on the body of the subject. Examples of an attachment include, but are not limited to, a snap-fit attachment configured to hold the wearable device in place using a snap-fit mechanism (for example, where the wearable device snap-fits into the attachment), an adhesive attachment configured to hold the wearable device in place using an adhesive, a magnetic attachment configured to hold the wearable device in place using magnets (for example, where the wearable device and attachment each comprise magnets), or other mechanical attachments (such as hook-and-loop fasteners, Velcro, indents and protrusions, or similar), or any other attachment, or any combination of attachments. However, while examples have been provided for the type of attachment, it will be understood that any attachment suitable to hold the wearable device for placement of the wearable device on the body of the subject can be used.

The apparatus 100 can comprise an attachment for a single wearable device or an attachment for multiple wearable devices. In some embodiments, the apparatus 100 itself can comprise the attachment or a cover (or case) of the apparatus 100 can comprise the attachment. The cover may remain on the apparatus 100 during everyday use. Since the attachment is configured to hold the wearable device for placement, the apparatus 100 can thus itself be used to move the wearable device toward the body of the subject for placement. In effect, the apparatus 100 can serve as a wearable device applicator according to some embodiments. Where the apparatus 100 comprises a user interface 108, the attachment may be provided on the opposite side of the apparatus 100 as the user interface 108 such that the attachment does not obstruct or interfere with the user interface 108. For example, in embodiments where the apparatus 100 is a mobile device, the attachment may be provided on the front of the mobile device or, preferably the back of the mobile device not to obstruct the screen.

In embodiments where the apparatus 100 comprises an attachment configured to hold the wearable device for placement of the wearable device on the body of the subject, the processor 102 of the apparatus 100 may further be configured to recognise or detect the point at which the wearable device is at the identified body part of the subject where the wearable device is to be placed and may automatically release the wearable device from the attachment at this point. In embodiments where the attachment is a magnetic attachment, the pull of the magnets may force the wearable device to release from the attachment. In embodiments where the attachment is an adhesive attachment, the wearable device may be released when a certain pressure is applied to the wearable device against the identified body part and the attachment is subsequently moved away from the identified body part. Alternatively, in some embodiments, the wearable device may be manually released from the attachment when the wearable device is on the identified body part.

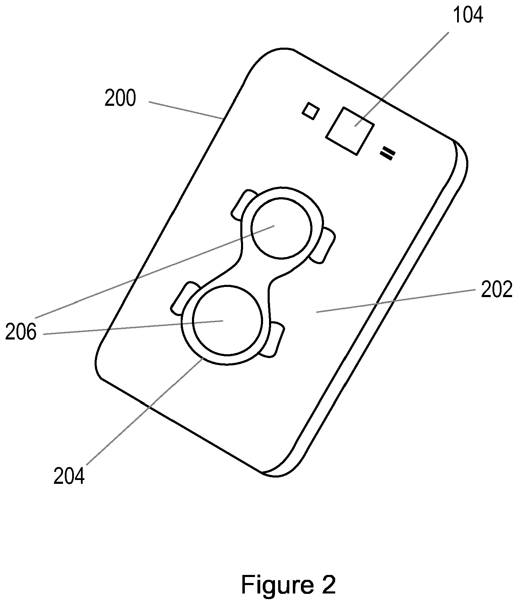

**Figure 2** illustrates an example of an apparatus 200 according to such an embodiment. In this illustrated example embodiment, the apparatus 200 is a mobile device and a back side 202 of the mobile device comprises an attachment 204 that is configured to hold one or more wearable devices 206 for use in placement of at least one of the one or more wearable devices 206 on the body of a subject. According to this example embodiment, the attachment 204 is configured to hold two wearable devices 206, where a first wearable device is positioned or arranged adjacent to a second wearable device. However, it will be understood that the attachment 204 illustrated in Figure 2 is merely one example and the attachment may alternatively be configured to hold any other number of wearable devices 206 in any other arrangement. In the example embodiment illustrated in Figure 2, the apparatus 200 also comprises a camera 104.

The camera 104 may be controllable by the processor of the apparatus 200 to acquire at least one image of the body of the subject for use in the method described herein.

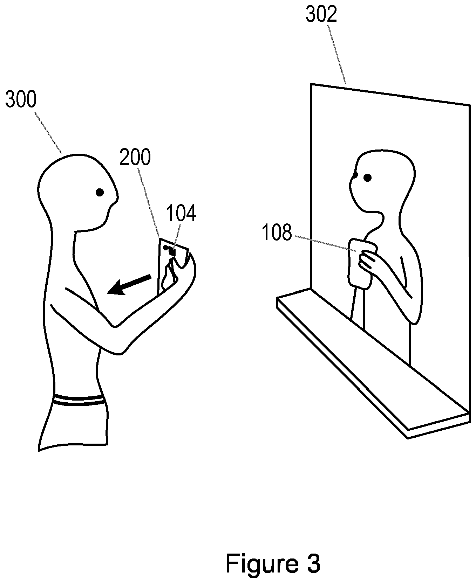

**Figure 3** illustrates an example of the apparatus 200 in use according to an embodiment. As illustrated in this example embodiment, the camera 104 of the apparatus 200 is aimed directly at the body of a subject 300 and a user interface 108 of the apparatus 200 is aimed indirectly at the subject 300 via a reflective surface 302 (which is a mirror in this example embodiment). In this way, a user of the apparatus 200, which is the subject 300 themselves in this example embodiment, can observe the guidance that is provided by the user interface 108 for wearable device placement via the reflective surface 302.

It will be understood that, in some embodiments, a wearable device can instead be moved independently of the apparatus 100 for wearable device placement. In these embodiments, the wearable device may comprise one or more markers (or distinctive features) and the processor 102 of the apparatus 100 can be configured to detect the one or more markers in the at least one acquired image for use in guiding placement of the wearable device on the body of a subject. In these embodiments, the one or more cameras 104 from which the at least one image is acquired may be sensitive to the one or more markers of the wearable device.

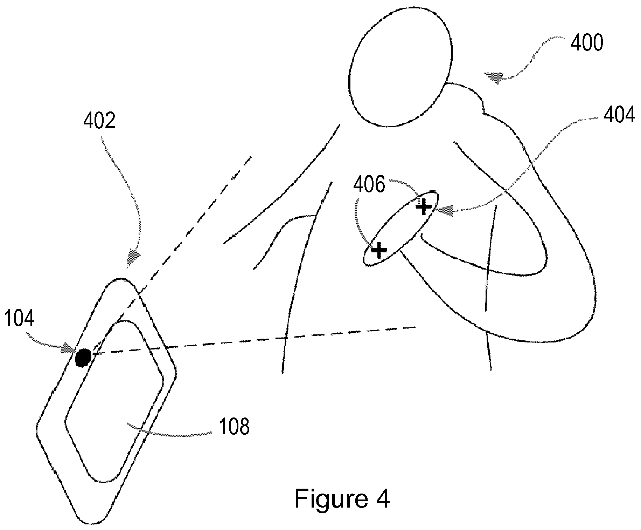

**Figure 4** illustrates an example of an apparatus 402 in use according to such an embodiment. In this illustrated example embodiment, the apparatus 402 is a mobile device comprising a camera 104 and a user interface 108. The camera 104 and the user interface 108 of the apparatus 402 are both aimed directly at the body of a subject 400 in this illustrated example embodiment. The subject 400 is in the field of view of the camera 104. The subject 400 moves a wearable device 404 independently of the apparatus 100 for placement of the wearable device 404 on their body. As illustrated, in this example embodiment, the wearable device 404 comprises a plurality (for example, two) markers 406 that the processor of the apparatus 402 can detect in the at least one image acquired by the camera 104 of the apparatus 402 for use in guiding placement of the wearable device 404 on the body of the subject 400. A user of the apparatus 402, which is the subject 400 themselves in this example embodiment, can directly observe the guidance that is provided by the user interface 108 of the apparatus 402 for wearable device placement.

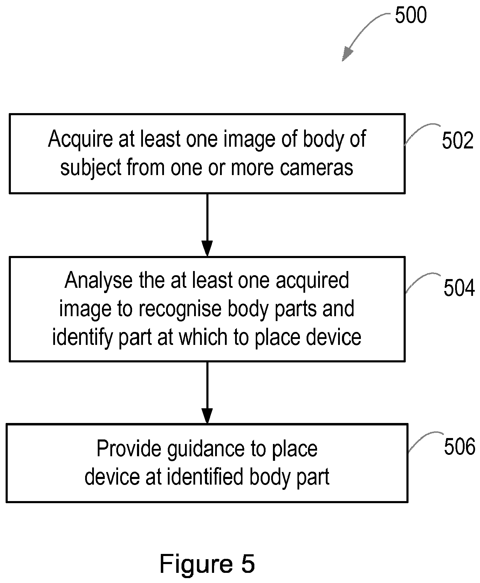

Figure 5 illustrates a method 500 of operating an apparatus comprising a processor to provide guidance for placement of a wearable device according to an embodiment. The illustrated method 500 can generally be performed by or under the control of the processor 102 of the apparatus 100.

With reference to Figure 5, at block 502, at least one image of the body of a subject is acquired from one or more cameras 104. As mentioned earlier, the processor 102 of the apparatus 100 can be configured to control the one or more cameras 104 to acquire at least one image of the body of the subject.

At block 504 of Figure 5, the at least one acquired image is analysed (or processed) to recognise body parts of the subject and to identify a body part of the subject at which to place the wearable device. The processor 102 of the apparatus 100 is configured to analyse (or process) the at least one acquired image in this way. In some embodiments, the body part at which to place the wearable device may be specific to a purpose for which the wearable device is dedicated. For example, the chest of the subject is specific to a wearable heart rate sensor. Alternatively, in some embodiments, the body part at which to place the wearable device may be predefined by a user of the apparatus 100 (such as the subject themselves, a medical professional, a carer, a family member, or any other user). For example, a user interface 108 may be configured to receive a user input defining the body part that is to be identified. Thus, the identified body part may be a body part that is preferred by the subject or another user and/or a body part that is correct or appropriate for the wearable device being placed on that body part.

The at least one acquired image may be analysed (or processed) to recognise body parts of the subject using any known recognition technique. For example, a skeleton recognition technique may be employed to recognise body parts of the subject in the at least one acquired image. In embodiments where the one or more cameras 104 comprise a front camera and a back camera of a mobile device, a skeleton recognition technique may be employed to recognise body parts of the subject in at least one image acquired from the front camera and at least one image acquired from the back camera. The results of the recognition can then be combined when identifying the body part of the subject at which to place the wearable device for a more accurate localisation of the identified body part. For example, where it is not possible (or no longer possible) to identify the body part in at least one image acquired from one of the cameras, it may be possible (or still be possible) to identify the body part in at least one image acquired from the other camera.

In some embodiments, the body part at which to place the wearable device may be automatically identified based on one or more (for example, generic) images of the body part stored in a memory 106 (which may be a memory 106 of the apparatus or a memory 106 external to the apparatus 100). For example, the body parts of the subject recognised in the at least one acquired image may be compared to the one or more images of the body part stored in the memory 106 in order to identify which of the recognised body parts to identify as the body part of the subject at which to place the wearable device.

Alternatively or in addition, the body part at which to place the wearable device may be identified based on a user input. For example, the body parts of the subject recognised in the at least one acquired image may be provided to the user and the user may provide an indication of one or more target body locations in the at least one acquired image at which to place the wearable device. In these embodiments, the processor 102 of the apparatus 100 may control a user interface 108 to provide the body parts of the subject recognised in the at least one acquired image to the user and the user may provide an indication of one or more target body locations in the at least one acquired image at which to place the wearable device via the same or a different user interface 108.

At block 506 of Figure 5, guidance is provided to place the wearable device at the identified body part of the subject. Specifically, the guidance provided guides placement of the wearable device toward the identified body part of the subject. The guidance provided can be indicative of the manner in which the wearable device needs to be moved for the wearable device to be placed at the identified body part of the subject. The guidance provided may, for example, include guidance on movements involving the position of the wearable device and/or the angle of the wearable device in relation to the identified body part of the subject.

In any of embodiments described herein, the processor 102 of the apparatus 100 may control one or more user interfaces 108, (which may be one or more user interfaces 108 of the apparatus 100, one or more user interfaces 108 external to the apparatus 100, or both) to provide (or render or output) the guidance. For example, the guidance may be provided by controlling any one or more of one or more lights on (or external) to the apparatus 100 to provide guidance, one or more speakers on (or external) to the apparatus 100 to provide guidance (for example, speech), one or more haptic feedback components on (or external) to the apparatus 100 to provide guidance (for example, vibrations), an augmented reality device external to apparatus 100 to provide the guidance (for example, by augmenting the guidance in three-dimensions when using augmented reality glasses), a smart device external to apparatus 100 to provide the guidance (for example, by augmenting the guidance on a camera image of the subject when using a smart device such as a smart mirror), a display on (or external) to the apparatus 100 to display the guidance, or any other user interfaces 108, or any combination of user interfaces 108 suitable to provide guidance.

In embodiments where a display screen is visible to a user guiding the wearable device, visual guidance may be provided on the display screen (such as by using arrows, signs colours and/or representations of the body part). Alternatively or in addition, audio guidance may be provided from one or more speakers, which may be useful where the display screen is not visible to the user.

In any of the embodiments described herein, the method may further comprise detecting a location of the wearable device in relation to the identified body part as the wearable device approaches the identified body part. The location of the wearable device may be recognised from the at least one acquired image. In these embodiments, the guidance provided to place the wearable device at the identified body part may comprise guidance to adjust the location of the wearable device in relation to the identified body part. Alternatively or in addition, in any of the embodiments described herein, the method may further comprise detecting an orientation of the wearable device in relation to the identified body part as the wearable device approaches the identified body part. The orientation of the wearable device may be recognised from the at least one acquired image. In these embodiments, the guidance provided to place the wearable device at the identified body part may comprise guidance to adjust the orientation of the wearable device in relation to the identified body part.

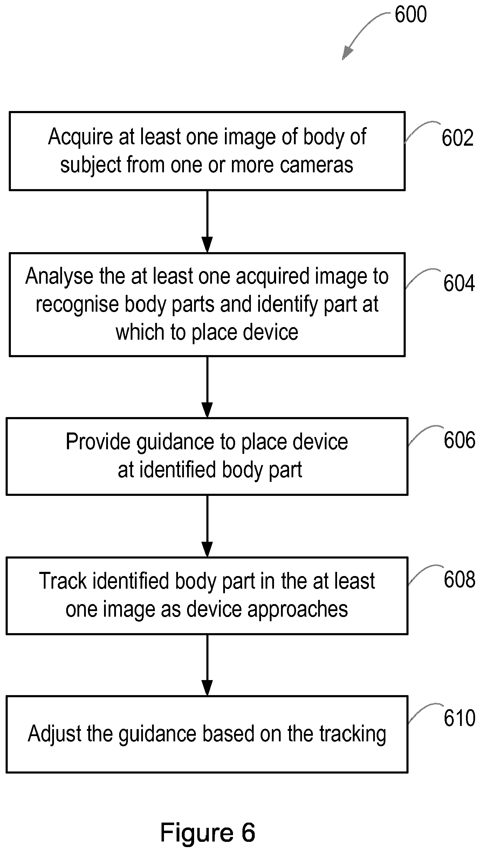

Figure 6 illustrates a method 600 of operating an apparatus comprising a processor to provide guidance for placement of a wearable device according to an example embodiment. The illustrated method 600 can generally be performed by or under the control of the processor 102 of the apparatus 100.

With reference to Figure 6, at least one image of the body of a subject is acquired from one or more cameras 104 (at block 602), the at least one acquired image is analysed to recognise body parts of the subject and to identify a body part of the subject at which to place the wearable device (at block 604), and guidance is provided to place the wearable device at the identified body part of the subject (at block 606). In other words, the method described above with reference to block 502, block 504 and block 506 of Figure 5 is performed and the corresponding description in respect of Figure 5 will be understood to also apply in respect of Figure 6, but will not be repeated here.

Then, at block 608 of Figure 6, the identified body part is tracked in the at least one image as the wearable device approaches the identified body part. For example, the identified body part may be tracked in or between subsequent or sequential images acquired from the one or more cameras 104 as the wearable device approaches the identified body part. The identified body part can be tracked using any body (or skeletal) tracking algorithm and a person skilled in the art will be aware of such algorithms. An example of a body (or skeletal) tracking algorithm includes an algorithm that tracks a body part based on kinematic and temporal information. However, it will be understood that any other body (or skeletal) tracking algorithm can be used. At block 610 of Figure 6, the guidance provided to place the wearable device at the identified body part is adjusted based on the tracking at block 608. In other words, the guidance provided to place the wearable device at the identified body part is adjusted based on the tracked identified body part.

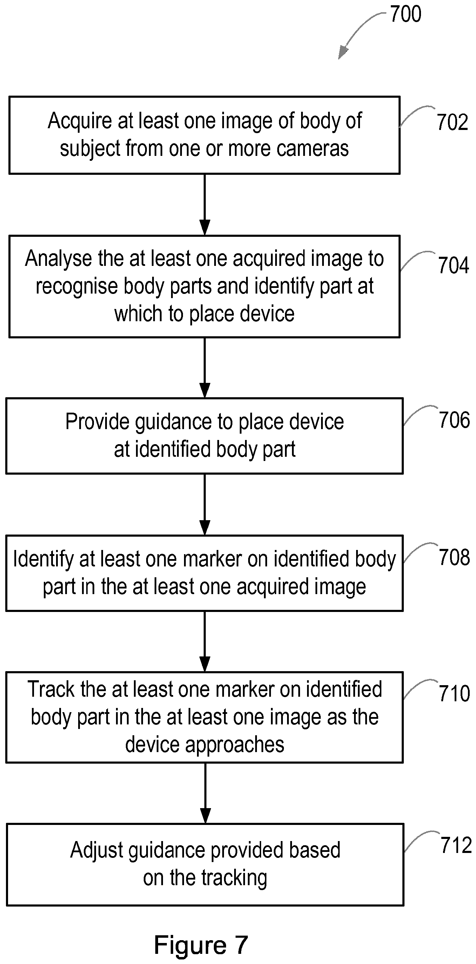

**Figure 7** illustrates a method 700 of operating an apparatus comprising a processor to provide guidance for placement of a wearable device according to another example embodiment. The illustrated method 700 can generally be performed by or under the control of the processor 102 of the apparatus 100.

With reference to Figure 7, at least one image of the body of a subject is acquired from one or more cameras 104 (at block 702), the at least one acquired image is analysed to recognise body parts of the subject and to identify a body part of the subject at which to place the wearable device (at block 704), and guidance is provided to place the wearable device at the identified body part of the subject (at block 706). In other words, the method described above with reference to block 502, block 504 and block 506 of Figure 5 is performed and the corresponding description in respect of Figure 5 will be understood to also apply in respect of Figure 7, but will not be repeated here.

Then, at block 708 of Figure 7, at least one marker (specifically, a body marker) is identified on the identified body part in the at least one acquired image. The at least one marker may, for example, comprise one or more skin features (such as a pore pattern, a pore distribution, a skin fold, a skin spot, a skin spot pattern, a birthmark, a local skin tone, a shadow cast on the skin from one or more bones, or any other skin feature, or any combination of skin features), one or more hair features (such as a hair density, a hair direction, a hairline, or any other hair feature, or any combination of hair features), one or more body features (such as a nipple, a nail, or any other body feature, or any combination of body features), or any other markers, or any combinations of markers.

In some embodiments, markers on the body of the subject may be set in an initial calibration phase. For example, at least one image of the body of the subject may be acquired in an initial calibration phase and a user may indicate markers on the body of the subject such that the indicated markers can subsequently be used (at block 708 of Figure 7) to identify at least one marker in the at least one acquired image. Alternatively or in addition, markers on the body of the subject may be detected during removal of a previously placed wearable device from the body of the subject and the detected markers can subsequently be used (at block 708 of Figure 7) to identify at least one marker in the at least one acquired image. At block 708 of Figure 7, the at least one marker may be identified in the at least one acquired image using any suitable feature detection (or feature recognition) technique and a person skilled in the art will be aware of such techniques.

At block 710, the at least one marker on the identified body part is tracked in the at least one image as the wearable device approaches the identified body part. For example, the at least one marker may be tracked in or between subsequent or sequential images acquired from the one or more cameras 104 as the wearable device approaches the identified body part. The at least one marker on the identified body part may be tracked in the at least one image using any suitable feature tracking technique and a person skilled in the art will be aware of such techniques.

At block 712, the guidance provided to place the wearable device at the identified body part is adjusted based on the tracking at block 710. In other words, the guidance provided to place the wearable device at the identified body part is adjusted based on the tracked at least one marker on identified body part.

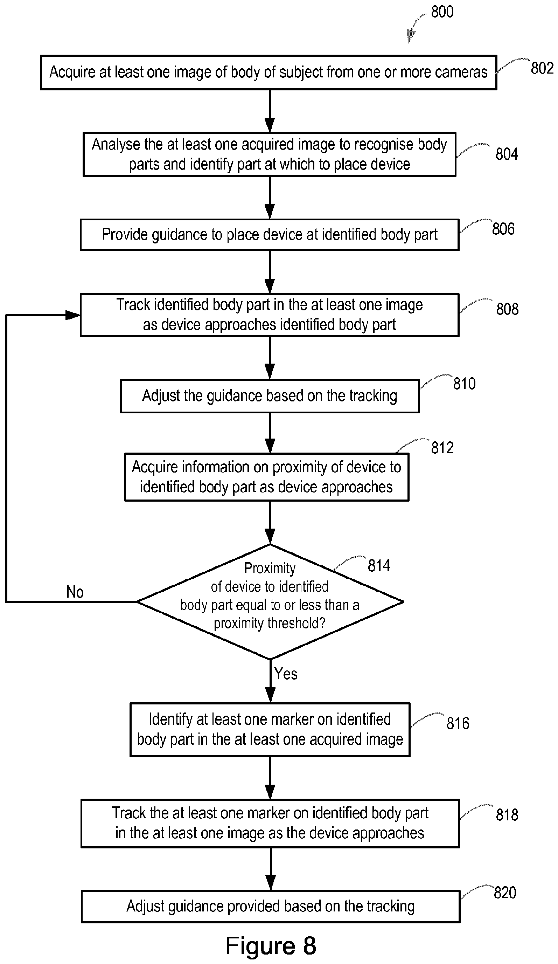

**Figure 8** illustrates a method 800 of operating an apparatus comprising a processor to provide guidance for placement of a wearable device according to another example embodiment. The illustrated method 800 can generally be performed by or under the control of the processor 102 of the apparatus 100.

With reference to Figure 8, at least one image of the body of a subject is acquired from one or more cameras 104 (at block 802), the at least one acquired image is analysed to recognise body parts of the subject and to identify a body part of the subject at which to place the wearable device (at block 804), and guidance is provided to place the wearable device at the identified body part of the subject (at block 806). In other words, the method described above with reference to block 502, block 504 and block 506 of Figure 5 is performed and the corresponding description in respect of Figure 5 will be understood to also apply in respect of Figure 8, but will not be repeated here.

Then, at block 808 of Figure 8, the identified body part is tracked in the at least one image as the wearable device approaches the identified body part. At block 810 of Figure 8, the guidance provided to place the wearable device at the identified body part is adjusted based on the tracking at block 808. In other words, the guidance provided to place the wearable device at the identified body part is adjusted based on the tracked identified body part. Thus, the method described above with reference to block 608 and block 610 of Figure 6 is performed and the corresponding description in respect of Figure 6 will be understood to also apply in respect of Figure 8, but will not be repeated here.

At block 812 of Figure 8, information on a proximity of the wearable device to the identified body part is acquired as the wearable device approaches the identified body part. For example, information on the proximity of the wearable device to the identified body part (and also recognition or detection of the point at which the wearable device is on the identified body part) can be acquired based on three-dimensional depth sensing (such as by using dual camera disparity information, a shadow depth from a flash, or infra-red time of flight techniques), a changing size or scale of the identified body part in the at least one acquired image, infra-red proximity sensing, or the covering (or partial covering) of the camera 104 or an ambient light sensor by the identified body part.

At block 814 of Figure 8, it is determined whether the proximity of the wearable device to the identified body part is equal to or less than (i.e. has reached or is within) a proximity threshold. When the proximity of the wearable device to the identified body part is greater than (i.e. has not reached or is outside) the proximity threshold, the identified body part continues to be tracked in the at least one image as the wearable device approaches the identified body part. The tracking of the identified body part continues until it is determined that the proximity of the wearable device to the identified body part is equal to or less than the proximity threshold. More specifically, the method described with respect to block 808, block 810, block 812, and 814 is repeated until it is determined that the proximity of the wearable device to the identified body part is equal to or less than the proximity threshold.

When the proximity of the wearable device to the identified body part is equal to or less than the proximity threshold, the method proceeds to block 816, where at least one marker is identified on the identified body part in the at least one acquired image. Then, at block 818, the at least one marker on identified body part is tracked in the at least one image as the wearable device approaches the identified body part. In other words, the method described above with reference to block 708 and block 710 of Figure 7 is performed and the corresponding description in respect of Figure 7 will be understood to also apply in respect of Figure 8, but will not be repeated here.

In some embodiments, the proximity threshold described above may be based on the field of view of the one or more cameras 104 from which the at least one image of the body of a subject is acquired. For example, in these embodiments, determining whether the proximity of the wearable device to the identified body part is equal to or less than a proximity threshold may comprise determining whether one or more reference features of the body of the subject are within (or at least partially within) the field of view of at least one of the cameras 104.

When the one or more reference features of the body are within (or at least partially within) the field of view of at least one of the cameras 104, it is determined that the proximity of the wearable device to the identified body part is greater than the proximity threshold and thus the identified body part continues to be tracked in the at least one image as the wearable device approaches the identified body part. Similarly, when the one or more reference features of the body are outside (or at least partially outside) the field of view of at least one of the cameras 104, it is determined that the proximity of the wearable device to the identified body part is equal to or less than the proximity threshold and thus at least one marker is identified on the identified body part and tracked in the at least one image as the wearable device approaches the identified body part. The reference features of the body can, for example, be any features in the vicinity of (for example, adjacent to) the identified body part. Examples of reference features of the body include, but are not limited to, a body part aside from the identified body part, a joint, an armpit, a bone (such as a collarbone), a marker on the body (such as any of those mentioned earlier), or any other reference feature of the body, or any combination of reference features.

The transition from tracking the identified body part itself to tracking at least one marker on the identified body part can be useful where it is no longer possible to identify the body part itself due to a closer range of the camera 104 to the identified body part that will occur according to some embodiments (for example, embodiments where the wearable device itself comprises the camera 104, embodiments where the apparatus 100 or another device used to move the wearable device comprises the camera 104, or any other embodiments where the camera moves toward the identified body part during wearable device placement).

At block 820 of Figure 8, the guidance provided to place the wearable device at the identified body part is adjusted based on the tracking at block 818. In other words, the guidance provided to place the wearable device at the identified body part is adjusted based on the tracked at least one marker on identified body part. Thus, the method described above with reference to block 712 of Figure 7 is performed and the corresponding description in respect of Figure 7 will be understood to also apply in respect of Figure 8, but will not be repeated here.

Therefore, there is provided herein an improved method and apparatus for providing guidance for placement of a wearable device. According to the method and apparatus described herein, it is possible to simply and accurately facilitate placement of a wearable device at a correct location on the body of a subject, irrespective of the unique anatomy of the subject. Furthermore, a more integrated system is provided (for example, where the wearable device and camera are integrated, or where the wearable device, wearable device attachment and camera are integrated) such that physical markers do not need to be attached to the body of the subject.

The method and apparatus described herein can be particularly useful in low acuity settings (such as in a general ward or at home) to support untrained users, including the subjects themselves, to routinely replace wearable devices without intervention or support from a medical professional. The method and apparatus described herein can be applied to, for example, wearable health monitoring devices such as wearable sensors (including electrocardiography ECG sensors, photoplethysmography PPG sensors and ultrasound sensors), wearable medication dispensers such as wearable patches for topical dispensing of medication, and medical hand-held devices (such as stethoscopes and ultrasound devices).

There is also provided a computer program product comprising a computer readable medium, the computer readable medium having computer readable code embodied therein, the computer readable code being configured such that, on execution by a suitable computer or processor, the computer or processor is caused to perform the method or methods described herein.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method (500, 600, 700, 800) of operating an apparatus comprising a processor to provide guidance for placement of a wearable device, the method comprising:
acquiring (502, 602, 702, 802) at least one image of the body of a subject from one or more cameras;
analysing (504, 604, 704, 804) the at least one acquired image to recognise body parts of the subject and to identify a body part of the subject at which to place the wearable device; and
providing (506, 607, 706, 806) guidance to place the wearable device at the identified body part of the subject.

2. A method as claimed in claim 1, wherein the identified body part is specific to a purpose for which the wearable device is dedicated.

3. A method as claimed in any one of the preceding claims, wherein the identified body part is a body part that is predefined by a user.

4. A method as claimed in any one of the preceding claims, wherein the body part at which to place the wearable device is identified using a skeleton recognition technique.

5. A method as claimed in any one of the preceding claims, the method further comprising:
tracking (608) the identified body part in the at least one image as the wearable device approaches the identified body part; and
adjusting (610) the guidance provided based on the tracking.

6. A method as claimed in any one of the preceding claims, the method further comprising:
detecting a location of the wearable device in relation to the identified body part as the wearable device approaches the identified body part; and
wherein the guidance provided to place the wearable device at the identified body part comprises guidance to adjust the location of the wearable device in relation to the identified body part.

7. A method as claimed in any one of the preceding claims, the method further comprising:
detecting an orientation of the wearable device in relation to the identified body part as the wearable device approaches the identified body part; and
wherein the guidance provided to place the wearable device at the identified body part comprises guidance to adjust the orientation of the wearable device in relation to the identified body part.

8. A method as claimed in any one of the preceding claims, the method further comprising:
acquiring (812) information on a proximity of the wearable device to the identified body part as the wearable device approaches the identified body part.

9. A method as claimed in claim 8, the method further comprising:
when (814) the proximity of the wearable device to the identified body part is equal to or less than a proximity threshold:
identifying (816) at least one marker on the identified body part in the at least one acquired image;
tracking (818) the at least one marker on identified body part in the at least one image as the wearable device approaches the identified body part; and
adjusting (820) the guidance provided based on the tracking.

10. A computer program product comprising a computer readable medium, the computer readable medium having computer readable code embodied therein, the computer readable code being configured such that, on execution by a suitable computer or processor, the computer or processor is caused to perform the method of any one of claims 1-9.

11. An apparatus (100) for providing guidance for placement of a wearable device, the apparatus (100) comprising:
a processor (102) configured to:
acquire at least one image of the body of the subject from one or more cameras (104);
analyse the at least one acquired image to recognise body parts of the subject and to identify a body part of the subject at which to place the wearable device; and
provide guidance to place the wearable device at the identified body part of the subject.

12. An apparatus (100) as claimed in claim 11, wherein one or more cameras (104) are aimed directly at the body of the subject, one or more cameras (104) are aimed indirectly at the body of the subject via a reflective surface, or one or more cameras (104) are aimed directly at the body of the subject and one or more cameras (104) are aimed indirectly at the body of the subject via a reflective surface.

13. An apparatus (100) as claimed in any one of claims 11 or 12, wherein:
the wearable device comprises at least one of the one or more cameras; or
a mobile device comprises at least one of the one or more cameras.

14. An apparatus (100) as claimed in claim 13, wherein the mobile device comprises an attachment configured to hold the wearable device for the placement.

15. An apparatus (100) as claimed in any one of claims 11-14, wherein the processor (102) is configured to control a user interface (108) to provide the guidance.
